# EUROPÄISCHE PATENTSCHRIFT

(11) **EP 0 655 230 B1**
(45) Veröffentlichungstag und Bekanntmachung des Hinweises auf die Patenterteilung: **15.09.1999**
(21) Anmeldenummer: 94117984.8
(22) Anmeldetag: 15.11.1994
(51) Int. Cl.: A61F 2/34

(54) **Konische Hüftgelenkpfanne ohne Selbsthemmung**
Conical acetabular cup without any self-blocking
Cupule cotyloidienne sans autoserrage

(30) Priorität: 26.11.1993 DE 4340304; 29.01.1994 DE 4402675
(43) Veröffentlichungstag der Anmeldung: 31.05.1995
(73) Patentinhaber: CERASIV GmbH INNOVATIVES KERAMIK-ENGINEERING, D-73207 Plochingen (DE)
(72) Erfinder: Pfaff, Hans-Georg, Dipl.-Ing. FH, D-73760 Ostfildern (DE); Kälberer, Hartmut, Dipl.-Ing. BA, D-73779 Deizisau (DE); Hoch, Ernst, D-73274 Notzingen (DE)
(74) Vertreter: Schulz, Wilfried, Dipl.-Ing.

(56) Entgegenhaltungen:
- EP-A- 0 123 514
- EP-A- 0 277 511
- WO-A-86/02261
- FR-A- 2 242 065
- US-A- 5 092 897

## Beschreibung

Die Erfindung betrifft eine Hüftgelenkpfanne

Hüftgelenk-Endoprothesen bestehen aus einer Hüftgelenkpfanne, die im Beckenknochen verankert ist und aus einer Kugel, die in die Pfanne drehbar eingesetzt ist und mit einem Schaft im Oberschenkelknochen verankert ist.

Hüftgelenkpfannen bestehen aus einer äußeren Metallschale, welche die Implantataußenkontur darstellt und aus einer inneren Gleitschale, die aus Keramik oder aus Kunststoff (UHMWPE = Ultra High Molekular Weight Polyethylen) hergestellt ist.

Der Begriff Metallschale steht synonym für den metallischen Außenteil des im Beckenknochen verankerten Implantates. Die Außenkontur ist den medizinischen Anforderungen entsprechend gestaltet.

Es ist Stand der Technik, die innere Gleitschale in der Metallschale mit Hilfe einer konischen Klemmung zu fixieren. Der Winkel der konischen Klemmung liegt bei 5° 43', d.h. einem Winkelverhältnis von 1:10. Die Gleitschale ist dabei selbsthemmend bzw. klemmend in die Metallschale eingesetzt. Es sind keine weiteren Befestigungsmittel vorgesehen.

Nachteilig hieran ist, daß die Gleitschale sich beim Einsetzen in die Metallschale leicht verkantet. Dadurch entsteht eine ungleichmäßige Kräfteverteilung, die unter Umständen zum Bruch der Gleitschale führen kann, insbesondere, wenn sie aus Keramik hergestellt ist.

Ein weiterer wesentlicher Nachteil ist, daß nach dem Einfügen der Gleitschale bzw. des Pfanneneinsatzes aufgrund der hohen Klemmkräfte die Gleitschale nicht mehr zerstörungsfrei entfernt werden kann. Die ist jedoch für den Operateur äußerst wichtig.

Ein weiterer Nachteil ist, daß bei einer konischen Klemmung von 1:10 der konvex geformte Teil der Gleitschale hohen Zugspannungen ausgesetzt ist, was zur Folge hat, daß die Gleitschale mit großen Wandstärken ausgeführt werden muß. Aus medizinischer Sicht besteht aber die Forderung nach kleinen Implantaten.

Aus der den Stand der Technik bildenden EP-A1-0 277 511 ist eine Hüftgelenkpfanne zum Einsetzen in Knochengewebe bekannt, die aus einer metallischen Außenpfanne und einem inneren Pfannenkörper aus Kunststoff besteht. Der Pfannenkörper ist dabei zur Außenpfanne hin von einem metallischen Mantel eng anliegend eingefaßt, wobei der aus Pfannenkörper und Mantel gebildete Einsatz mit mindestens einem Befestigungselement eng anliegend in der Außenpfanne verankert ist. Die Auflagefläche der Außenpfanne ist konisch ausgebildet, wobei der Einsatz klemmfrei eingesetzt ist, d. h. eine Klemmkonus-Wirkung ist verhindert. Zur Befestigung des Pfannenkörpers ist ein Haltering vorgesehen, der sich über den Innenrand der Außenpfanne erstreckt. Außerdem kann die Befestigung auch über einen Spannring erfolgen.

Der Erfindung liegt die Aufgabe zugrunde, eine Hüftgelenkpfanne zum Einsetzen in Knochengewebe derart zu verbessern, daß der Pfanneneinsatz bzw. die Gleitschale gegen Verdrehen und Herausfallen geschützt ist und sich zerstörungsfrei aus ihrem Sitz herausdrücken und auswechseln läßt und gleichzeitig eine günstigere Belastungssituation der Gleitschale bewirkt wird.

Erfindungsgemäß wird diese Aufgabe in einer bevorzugten Ausführungsform durch die Mermale des Anspruchs 1 und in einer alternativen Ausführungsform durch die Merkmale des Anspruchs 2 gelöst.

Dadurch, daß die Gleitschale ohne Selbsthemmung auf der konischen Auflagefläche aufliegt, läßt sie sich einfach auswechseln. Dies ist für den Operateur extrem wichtig.

Ohne Selbsthemmung bedeutet, daß die Gleitschale auf ihrem Sitz nicht eingeklemmt ist, sondern lediglich aufsitzt.

Die Verankerung der Gleitschale in der Metallschale erfolgt in bevorzugter Ausführungsform über einen Haltering, der alleine die Festlegung der Gleitschale bewirkt.

Erfindungsgemäß liegt der Winkel der konischen Auflagefläche zwischen 20° und 90°, in bevorzugter Ausführungsform bei 40°.

Erfindungsgemäß ist der Haltering auf der Stirnseite der Metallschale mittels Schrauben befestigt und erstreckt sich über den Innenrand der Metallschale hinaus. Dieser sich über den Innenrand hinaus erstreckende Bereich des Halterings dient als Anlagefläche für die Gleitschale.

Erfindungsgemäß ist die Gleitschale aus Keramik hergestellt.

In beiden Ausführungsformen ist unterhalb der konischen Auflagefläche zwischen der Gleitschale und dem Boden der Metallschale ein Freiraum angeordnet, wodurch die Gleitschale aus Keramik nicht extrem paßgenau gefertigt werden muß.

In der alternativen Ausführungsform weist der obere äußere Rand der Gleitschale eine nach Innen gerichtete konische Neigung auf. Die Gleitschale ist dabei über einen auf der konischen Neigung aufliegenden Spannring in der Metallschale verankert. In dieser Ausführungsform ist demnach der Haltering als Spannring ausgebildet.

Durch diese Maßnahmen ist eine hohe mechanische Festigkeit zu erreichen, da die tragende Fläche in Hauptlastrichtung orientiert ist. Hierdurch werden Zugspannungen vermieden und, da die Gleitschale aus Keramik besteht und damit eine außerordentlich hohe Druckfestigkeit besitzt, kann eine geringe Wandstärke und kleine Bauhöhe des gesamten Implantates ermöglicht werden.

Zweckmäßigerweise liegen die obere Stirnseite der Metallschale, des Spannringes und der Metallschale auf gleicher Höhe. Hierdurch weist die Hüftgelenkpfanne eine kompakte Bauform auf.

In einer ersten alternativen Ausführungsform ist der Spannring über ein Gewinde in der Metallschale eingeschraubt.

In einer zweiten vorteilhaften Ausführungsform weist der Spannring Bohrungen auf und ist über Schrauben in der Metallschale festgelegt.

Beide alternativen Ausführungsformen ermöglichen eine spaltfreie Montage der Gleitschale in der Metallschale. Weiterhin ist durch den Spannring der Rand der Gleitschale unter Druckspannung gesetzt, was sich ebenfalls günstig auf die mechanische Belastbarkeit des Implantates auswirkt.

Erfindungsgemaß erstreckt sich vorteilhafterweise die konische Auflagefläche der Gleitschale vom Spannring bzw. Haltering bis annähernd zum Boden der Metallschale.

Nachfolgend wird die Erfindung anhand von vier Figuren näher erläutert.

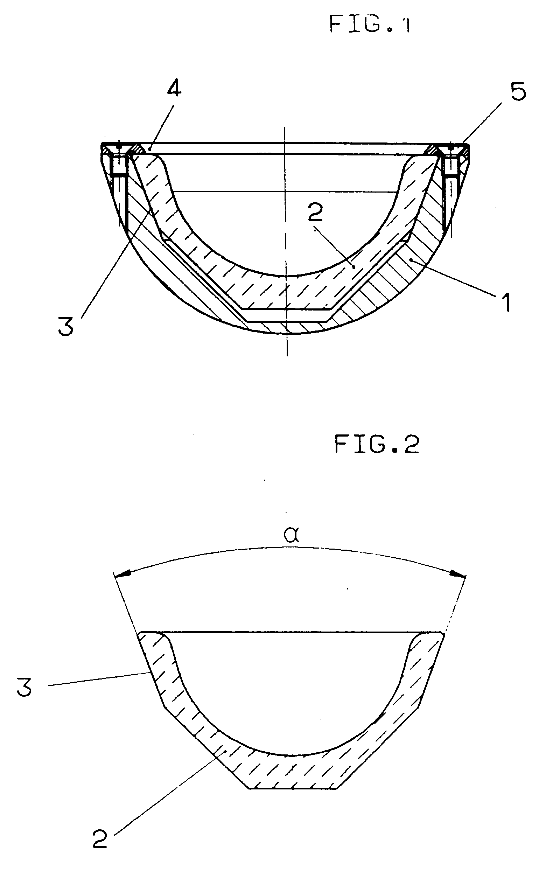

Figur 1 zeigt eine erfindungsgemäße Hüftgelenkpfanne, die aus einer äußeren Metallschale 1 und einer inneren Gleitschale 2 aus Keramik besteht. Die Metallschale 1 ist aus Titan gefertigt. Sie bildet die Implantataußenkontur und wird in das Knochengewebe eingesetzt. Die Oberkanten der Metallschale 1 und Gleitschale 2 liegen auf derselben Höhe.

Die Metallschale 1 ist in ihrem Inneren mit einer konischen Auflagefläche 3 für die Gleitschale 2 versehen. Die Auflagefläche 3 erstreckt sich auf der gesamten Umfangsfläche der Metallschale 1. Die Gleitschale 2 hat eine hieran angepaßte Auflagefläche 3.

Erfindungsgemäß sitzt die Gleitschale 2 ohne Selbsthemmung auf der konischen Auflagefläche 3 auf. Der Winkel der konischen Auflagefläche 3 muß daher so groß gewählt werden, daß keine Klemmung oder Selbsthemmung auftritt. Dies ist auf jeden Fall bei einem Winkel α von 20° bis 90° der Fall. Bevorzugt ist ein Winkel α von um die 40°.

Figur 2 zeigt im Schnitt eine Gleitschale 2 mit denselben Abmessungen wie in Figur 1. Sehr gut ist die Konizität der Auflagefläche 3 mit dem Winkel α zu sehen.

In Figur 1 ist ein Haltering 4 gezeigt, mit dem die Gleitschale 2 in der Metallschale 1 verankert ist. Der Haltering 4 ist auf der Stirnseite der Metallschale 1 mit Schrauben 5 befestigt und erstreckt sich über den Innenrand der Metallschale 1 hinaus. Dieser Bereich dient als Anlagefläche für den oberen Rand der Gleitschale 2.

Mit der erfindungsgemäßen Hüftglenkpfanne ist eine leichte Auswechselbarkeit der Gleitschale 2 bei gleichzeitiger sicherer Verankerung in der Metallschale 1 gewährleistet.

Fig. 3 zeigt eine Ausführungsform einer Hüftgelenkpfanne, ebenfalls mit einer Metallschale 1 aus Titan und einer Gleitschale 2 aus Keramik, z.B. Aluminiumoxid oder eine ihrer Legierungen.

Der obere äußere Rand der Gleitschale 2 ist in dieser Ausführungsform mit einer nach Innen gerichteten konischen Neigung 6 versehen. Diese konische Neigung 6 ist entgegengesetzt der konischen Auflagefläche 3 gerichtet und grenzt an diese an. Verankert ist die Gleitschale 2 über einen Spannring 7 als Haltering, der auf der konischen Neigung 6 aufliegt und die Gleitschale 2 auf die Metallschale 1 drückt.

Die Stirnseite der Metallschale 1, des Spannringes 7 und der Gleitschale 2 liegen auf derselben Höhe. An der äußeren Umfangsfläche des Spannringes 7 ist dieser bzw. die angrenzende Metallschale 1 mit einem Gewinde 8, bevorzugt ein Feingewinde, versehen. Der Spannring 7 läßt sich somit leicht eindrehen und drückt dabei die Gleitschale 2 auf die Metallschale 1. Zum leichteren Einschrauben des Spannringes 7 können z.B. auf seiner Stirnseite Vertiefungen als Eingriff für ein Werkzeug angeordnet sein.

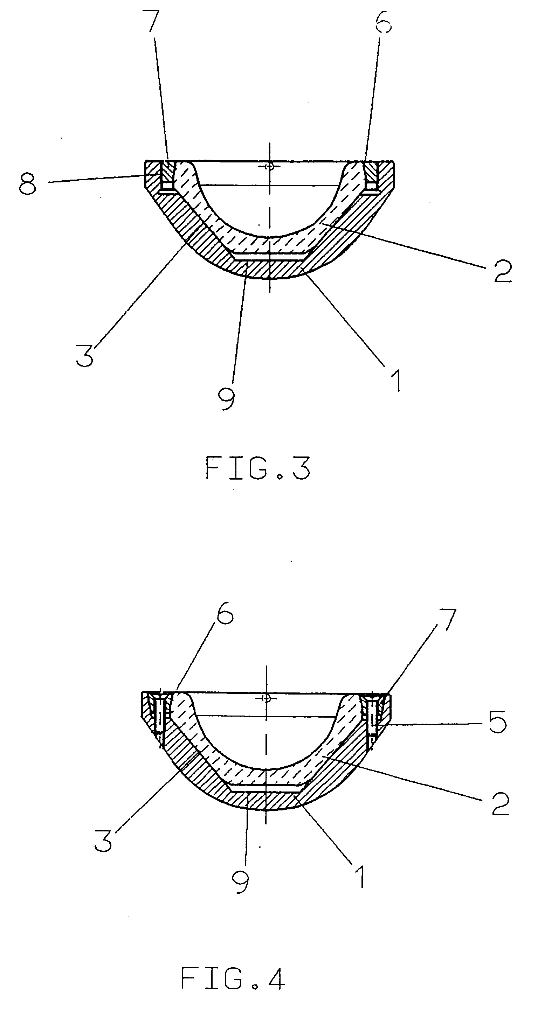

Fig. 4 zeigt eine Ausführungsform, die der der Fig. 3 sehr ähnelt. Jedoch weist der Spannring 7 hier Bohrungen auf, über die der Spannring 7 mittels Schrauben 5 an der Metallschale 1 festgelegt ist. Ansonsten ist diese Ausführungsform mit der der Fig. 3 identisch. Gleiche Bezugsziffern zeigen auch den gleichen Gegenstand.

Den Ausführungsformen, die in den Figuren 3 und 4 gezeigt sind, ist beiden gemeinsam, daß sich die konische Auflagefläche 3 der Gleitschale 2 vom Spannring 7 bis annähernd zum Boden 9 der Metallschale 1 erstreckt. Dieses Merkmal kann auch vorteilhafterweise bei den Ausführungsformen gemäß der Figuren 1 und 2 vorgesehen werden.

## Patentansprüche

1. Hüftgelenkpfanne zum Einsetzen in Knochengewebe bestehend aus einer äußeren Metallschale (1) und einer inneren Gleitschale (2) aus Keramik, wobei die Auflagefläche (3) der Gleitschale (2) in der Metallschale (1) konisch ausgebildet ist, die Gleitschale (2) ohne Selbsthemmung auf der konischen Auflagefläche (3) aufliegt und über einen Haltering (4) in der Metallschale (1) fixiert ist, der Haltering (4) auf der Stirnseite der Metallschale (1) mittels Schrauben (5) befestigt ist und sich über den Innenrand der Metallschale (1) hinaus erstreckt und unterhalb der konischen Auflagefläche (3) zwischen der Gleitschale (2) und dem Boden (9) der Metallschale (1) ein Freiraum angeordnet ist.

2. Hüftgelenkpfanne zum Einsetzen in Knochengewebe bestehend aus einer äußeren Metallschale (1) und einer inneren Gleitschale (2) aus Keramik, wobei die Auflagefläche (3) der Gleitschale (2) in der Metallschale (1) konisch ausgebildet ist, die Gleitschale (2) ohne Selbsthemmung auf der konischen Auflagefläche (3) aufliegt und über einen Haltering (4) in der Metallschale (1) fixiert ist, der obere äußere Rand der Gleitschale (2) eine nach innen gerichtete konische Neigung (6) aufweist und die Gleitschale (2) über einen auf der konischen Neigung (6) aufliegenden Spannring (7) als Haltering in der Metallschale verankert ist und unterhalb der konischen Auflagefläche (3) zwischen der Gleitschale (2) und dem Boden (9) der Metallschale (1) ein Freiraum angeordnet ist.

3. Hüftgelenkpfanne nach Anspruch 1 oder 2, **dadurch gekennzeichnet**, daß der Winkel (α) der konischen Auflagefläche (3) zwischen 20° und 90° liegt.

4. Hüftgelenkpfanne nach einem der Ansprüche 1 bis 3, **dadurch gekennzeichnet**, daß der Winkel (α) der konischen Auflagefläche (3) bei 40° liegt.

5. Hüftgelenkpfanne nach einem der Ansprüche 2 bis 4, **dadurch gekennzeichnet,** daß die obere Stirnseite der Metallschale (1), des Spannrings (7) und der Gleitschale (2) auf gleicher Höhe liegen.

6. Hüftgelenkpfanne nach einem der Ansprüche 2 bis 5, **dadurch gekennzeichnet,** daß der Spannring (7) über ein Gewinde (8) in der Metallschale (1) eingeschraubt ist.

7. Hüftgelenkpfanne nach einem der Ansprüche 2 bis 6, **dadurch gekennzeichnet** daß der Spannring (7) Bohrungen aufweist und über Schrauben (5) in der Metallschale (1) festgelegt ist.

## Claims

1. Hip-joint socket for insertion in osseous tissue, comprising an outer metal shell (1) and an inner sliding shell (2) made of ceramics, wherein the bearing surface (3) of the sliding shell (2) in the metal shell (1) is conical in form, the sliding shell (2) rests without self-locking on the conical bearing surface (3) and is fixed in the metal shell (1) by way of a retaining ring (4), the retaining ring (4) is fastened on the end face of the metal shell (1) by means of screws (5) and extends beyond the inside edge of the metal shell (1), and, beneath the conical bearing surface (3), between the sliding shell (2) and the base (9) of the metal shell (1), is a clearance.

2. Hip-joint socket for insertion in osseous tissue, comprising an outer metal shell (1) and an inner sliding shell (2) made of ceramics, wherein the bearing surface (3) of the sliding shell (2) in the metal shell (1) is conical in form, the sliding shell (2) rests without self-locking on the conical bearing surface (3) and is fixed in the metal shell (1) by way of a retaining ring (4), the upper outer edge of the sliding shell (2) has an inwardly directed conical slope (6) and the sliding shell (2) is anchored in the metal shell by way of a clamping ring (7) as retaining ring that rests on the conical slope (6), and, beneath the conical bearing surface (3), between the sliding shell (2) and the base (9) of the metal shell (1), is a clearance.

3. Hip-joint socket according to claim 1 or 2, characterised in that the angle (α) of the conical bearing surface (3) is between 20° and 90°.

4. Hip-joint socket according to one of claims 1 to 3, characterised in that the angle (α) of the conical bearing surface (3) is 40°.

5. Hip-joint socket according to one of claims 2 to 4, characterised in that the upper end faces of the metal shell (1), the clamping ring (7) and the sliding shell (2) are at the same height.

6. Hip-joint socket according to one of claims 2 to 5, characterised in that the clamping ring (7) is screwed into the metal shell (1) by way of a thread (8).

7. Hip-joint socket according to one of claims 2 to 6, characterised in that the clamping ring (7) has bores and is fixed in the metal shell (1) by way of screws (5).

## Revendications

1. Cupule cotyloïdiennne destinée à être insérée dans un tissu osseux, comprenant une coquille extérieure (1) en métal et une coquille de glissement intérieure (2) en céramique, la surface d'appui (3) de la coquille de glissement (2) dans la coquille en métal (1) étant conformée en cône, la coquille de glissement (2) reposant sans autoserrage sur la surface d'appui (3) conique et étant immobilisée dans la coquille en métal (1) par un anneau de retenue (4), l'anneau de retenue (4) étant fixé au moyen de vis à la face frontale de la coquille en métal (1) et s'étendant au-delà du bord intérieur de la coquille en métal (1) et un espace libre étant disposé sous la surface d'appui (3) conique, entre la coquille de glissement (2) et le fond (9) de la coquille en métal (1).

2. Cupule cotyloïdiennne destinée à être insérée dans un tissu osseux, comprenant une coquille extérieure (1) en métal et une coquille de glissement intérieure (2) en céramique, la surface d'appui (3) de la coquille de glissement (2) dans la coquille en métal (1) étant conformée en cône, la coquille de glissement (2) reposant sans autoserrage sur la surface d'appui (3) conique et étant immobilisée dans la coquille en métal (1) par un anneau de retenue (4), le bord supérieur extérieur de la coquille de glissement (2) présentant inclinaison en cône (6) en direction de l'intérieur et la coquille de glissement (2) étant bloquée dans la coquille en métal par un anneau de serrage (7) reposant sur l'inclinaison en cône (6) comme anneau de retenue et un espace libre étant disposé sous la surface d'appui (3) conique, entre la coquille de glissement (2) et le fond (9) de la coquille en métal (1).

3. Cupule cotyloïdiennne selon la revendication 1 ou 2, caractérisée par le fait que l'angle (α) de la surface d'appui conique (3) est compris entre 20° et 90°.

4. Cupule cotyloïdiennne selon une des revendications 1 à 3, caractérisée par le fait que l'angle (α) de la surface d'appui conique (3) est d'environ 40°.

5. Cupule cotyloïdiennne selon une des revendications 2 à 4, caractérisée par le fait que les faces frontales supérieures de la coquille en métal (1), de l'anneau de serrage (7) et de la coquille de glissement (2) sont situées à la même hauteur.

6. Cupule cotyloïdiennne selon une des revendications 2 à 5, caractérisée par le fait que l'anneau de serrage (7) est vissé dans la coquille en métal (1) au moyen d'un filetage (8).

7. Cupule cotyloïdiennne selon une des revendications 2 à 6, caractérisée par le fait que l'anneau de serrage (7) comporte des trous et est fixé dans la coquille en métal (1) au moyen de vis (5).
